# EUROPÄISCHE PATENTANMELDUNG

(11) **EP 1 330 992 A1**
(43) Veröffentlichungstag der Anmeldung: **30.07.2003**
(21) Anmeldenummer: 02001583.0
(22) Anmeldetag: 23.01.2002
(51) Int. Cl.: A61B 19/00

(54) **Vorrichtung und Verfahren zur räumlichen Bestimmung einer Position eines Instrumentes an einem Gegenstand**

(71) Anmelder: Stiftung für Plastische und Aesthetische Wundheilung im Sondervermögen der DT Deutschen Stiftungstreuhend AG, 90762 Fürth (DE)
(72) Erfinder: Gehl, Dr. Gerolf, 8700 Küsnacht (CH)
(74) Vertreter: Patentanwälte Schaad, Balass, Menzl & Partner AG

(57) **Zusammenfassung**

Die vorliegende Erfindung betrifft eine Vorrichtung und ein Verfahren zur räumlichen Bestimmung einer Position eines Instrumentes an einem Gegenstand unter Bezugnahme auf dessen Gegenstück beziehungsweise Abbildung, enthaltend ein erstes Sensorsystem zur Erfassung der räumlichen Lage von drei ersten Fixpunkten (A,B,C) an dem Gegenstand, ein Instrument, ein zweites Sensorsystem zur räumlichen Erfassung der Position des Instrumentes und entweder Mittel zum Abbilden des Gegenstandes oder Mittel zur Erfassung der räumlichen Lage von drei zweiten Fixpunkten (X,Y,Z) am Gegenstück und einen Zeiger.

## Beschreibung

Die vorliegende Erfindung betrifft eine Vorrichtung zur räumlichen Bestimmung einer Position eines Instrumentes an einem Gegenstand unter Bezugnahme auf dessen Gegenstück beziehungsweise und ein entsprechendes Verfahren.

Heutzutage ist ein Ziel der Chirurgie mit möglichst geringen invasiven Mitteln chirurgische Manipulationen im menschlichen Körper vorzunehmen, z.B. beim Plazieren von Gelenkersatzteilen (Implantate), bei Tumorexzisionen (Ausräumungen von Tumoren im Körper), bei der Manipulationen im Neurocranium (Tumore etc.).

Bis zum jetzigen Zeitpunkt ist dabei immer noch eine direkte (Aufklappung) oder eine indirekte Sicht über eine Mikrokamera im Operationsgebiet notwendig.

Die Verwendung von eines Navigationssystems für die Lokalisation von Instrumenten in der Chirurgie ist bereits bekannt. Zur Eichung des Systems muss eine Ausgangsposition festgelegt werden, um den virtuellen Patienten auf dem Monitor mit dem echten auf dem Operationstisch in Deckung zu bringen. Dies erfolgt durch einen Referenzpunkt, welcher eindeutig am Patienten identifizierbar und auch im Datensatz auffindbar sein muss. Ein Problem ist dabei das permanente Registrieren der Patientenposition. Dies wird durch einen Leuchtdiodenstern erreicht, der fest mit dem Patientenkopf verbunden wird. In der Regel geschieht dies durch eine Metallklemme mit drei Stahldornen, in die der Patientenkopf eingespannt wird. Der so im Knochen verankerte Leuchtdiodenstern ist für den Operator im Wege, da zu monumental ist. Er lässt sich nicht verkleinern und neben dem Operationsfeld fixieren.

Ein grosser Nachteil aller gebräuchlicher Navigationssysteme liegt darin, dass das Referenzsystem nur an einem einzigen Punkt fixiert ist, was eine mangelnde Stabilität zur Folge hat. Diese mangelnde Stabilität kann zu durch Bewegungen während der Operation dazu führen, dass vitale Strukturen bedroht werden.

Aufgabe der vorliegenden Erfindung ist eine Vorrichtung zur räumlichen Bestimmung einer Position eines Instrumentes an einem Gegenstand, die unabhängig von Bewegungen des Gegenstandes ist.

Eine weitere Aufgabe der vorliegenden Erfindung ist ein Verfahren zur räumlichen Bestimmung einer Position eines Instrumentes an einem Gegenstand, die unabhängig von Bewegungen des Gegenstandes ist.

Die obigen Probleme werden gelöst durch eine Vorrichtung gemäss dem unabhängigen Anspruch 1 und ein Verfahren gemäss Anspruch 5. Bevorzugte Ausführungsformen sind in den abhängigen Ansprüchen beansprucht.

Die räumliche Bestimmung einer Position eines Instrumentes an einem Gegenstand wird erfindungsgemäss mit Bezugnahme auf das Gegenstück des Gegenstandes oder dessen Abbildung ausgeführt.

Unter einen Gegenstand wird in der vorliegenden Erfindung ein Teil des menschlichen Körpers verstanden, wie zum Beispiel Kopf, Bein, Rumpf usw. oder auch ein zu bearbeitendes Werkstück, wie zum Beispiel ein Werkstück zur Herstellung einer Prothese oder eines Implantates.

Unter dem Ausdruck Gegenstück des Gegenstandes werden Teile des menschlichen Körpers verstanden, für die beispielsweise eine Prothese erstellt werden soll.

Unter einer Abbildung des Gegenstandes wird eine Bilddateninformation verstanden, die beispielsweise durch Computertomographie, durch Kernspintomographie, durch Holographie, durch Sonographie oder durch andere dem Fachmann bekannte Methoden erhalten werden kann.

Unter dem Instrument werden chirurgische Instrumente, wie Sonden, Endoskope, Laser usw. verstanden, aber auch Instrumente, die bei der Herstellung von Prothesen und Implantaten verwendet werden, wie Sägen, Fräsen usw.

Am Gegenstand werden drei erste Fixpunkte (A,B,C) markiert. Die Markierung erfolgt vorzugsweise durch Sender, die Ultraschall-, IR- oder andere elektromagnetische Strahlung aussendend oder ein stationäres elektromagnetisches Feld erzeugen. Diese Markierungsmittel werden mittels eines ersten Sensorsystems in ihrer räumlichen Lage umfasst. Geeignete Sensorsysteme sind dem Fachmann bekannt. Vorzugsweise ist eine berührungslose und drahtlose Erfassung der an den Fixpunkten befestigten Sensoren über Kameras im Operationssaal möglich. Aufgrund dieser drei ersten Fixpunkte (A,B,C) werden drei zweite Fixpunkte (X,Y,Z) am dem Gegenstück beziehungsweise der Abbildung bestimmt, die den ersten Fixpunkten (A,B,C) entsprechen. Die Fixpunkte (A,B,C) bestimmen damit jeden Ort des Gegenstandes eindeutig und die Fixpunkte (X,Y,Z) bestimmen jeden Ort des Gegenstückes eindeutig.

Die Position des Instrumentes wird in seiner räumlichen Lage mittels eines zweiten Sensorssystems erfasst. Dazu muss ein Sender mit dem Instrument lagefest verbunden sein. Geeignete Sender sind solche, die Ultraschall-, IR- oder andere elektromagnetische Strahlung aussenden oder ein stationäres elektromagnetisches Feld erzeugen. Das zweite Sensorsystem kann die Strahlung beziehungsweise die elektromagnetischen Felder erfassen. Die so ermittelte räumliche Lage des Instrumentes entspricht einem Ort des Gegenstandes, der bezüglich der ersten Fixpunkte (A,B,C) bestimmt wird. Die so erhaltene Information über die räumliche Lage des Instrumentes wird an eine Bildverarbeitungseinheit übertragen. Das erste und das zweite Sensorsystem können in einer Ausführungsform das gleiche sein.

Das Mittel zum Abbilden des Gegenstandes bildet den Gegenstand, die den drei ersten Fixpunkten (A,B,C) entsprechenden Fixpunkte (X,Y,Z) und die durch das zweite Sensorsystem erfasste räumliche Position des Instrumentes ab. Dabei handelt es sich vorzugsweise um eine Bildverarbeitungseinheit, d.h. um einen Computer. Diese Bildverarbeitungseinheit zeigt Computertomographiebilder oder Kernspintomographiebilder über den zu therapierenden Körperbereich in dreidimensionaler Form. Nachdem relativ zu den Fixpunkten (A,B,C) die originalen Positionsdaten der Abbildung relativ zu den Fixpunkten (X,Y,Z) zugeordnet werden, ist eine lagerichtige Positionserkennung von Instrumenten im Bilddatensatz möglich.

Alternativ kann ein Mittel zur Erfassung der räumlichen Lage von drei zweiten Fixpunkten (X,Y,Z) am Gegenstück ein Bestandteil der Vorrichtung sein. Dies kann beispielsweise ein zweites Sensorsystem sein, aber es ist auch möglich das Gegenstück an einem fest befestigten Stativ zu fixieren. Ein Zeiger zeigt auf dem Gegenstück die räumliche Position des Instrumentes an dem Gegenstand an. Ein solcher Zeiger kann beispielsweise ein Laser oder ein Stift mit einer Spitze sein.

Ein besonderer Vorteil der erfindungsgemässen Vorrichtung und des erfindungsgemässen Verfahrens besteht darin, dass die Referenzfixierung nicht mehr an einem Punkt vorgenommen wird, sondern an drei weit voneinander entfernten Fixpunkten, die eine Fläche definieren. Idealerweise weisen die Fixpunkte voneinander einen minimalen Abstand von 3 bis 30 cm, vorzugsweise von 5 bis 20 cm auf. Der optimale Abstand der Fixpunkte hängt vom Manipulationsort ab. So ist der optimale Abstand der Fixpunkte bei einer Kopfoperation 7 bis 12 cm, bei einer Operation im Beckenbereich 20 bis 30 cm und für Manipulationen für Kronen und Brücken (Zahnchirurgie 3 bis 5 cm). Da die ersten Fixpunkte (A,B,C) vor der Erstellung der Abbildung festgelegt ist, können die zweiten Fixpunkte (X,Y,Z) sehr genau in die Abbildung übernommen werden.

Ein weiterer Vorteil der erfindungsgemässen Vorrichtung und des erfindungsgemässen Verfahrens besteht darin, dass die Sensoren zierlich dimensioniert sein können, da sie keine elektronische Konstruktion tragen müssen. Die Sensoren werden unabhängig voneinander an dem Gegenstand befestigt. Idealerweise findet eine Verschraubung dieser Sender statt, sodass die Fixpunkte (A,B,C) auch nach längeren Zeitpunkten wieder genau lokalisiert werden können. Dadurch können bei jedem neuen Eingriff die aktuellen Bilder mit früheren Dokumentationen kongruent übereinandergelegt werden und Veränderungen genau identifiziert werden. Dies ist auch eine hervorragende Methode, Veränderungen bei Tumoren, Entzündungen oder ähnlichen Vorgängen exakt zu verglichen (Vergrösserungen, Einschmelzungen).

Bei einer weiteren Ausführungsform wird die Manipulation modellhaft in der Holographie durchgeführt und aus dem Holographiebild werden die Kommandos für Laser oder andere geeignete Energien oder Gegenstände zum Schnitt, zum Brennen oder andere physikalisch wirksame Befehle (Impulse) im Gegenstand erteilt. So können zum Beispiel vom Hologramm aus ferngesteuerte Robotik-Raupen durch das Gewebe dringen und Wirkstoffe bis an den Einsatzort im Gewebe transportieren. Auf diese Weise können beispielsweise Medikamente in die Leber oder den Pankreas geführt werden oder auch Zellen aus bestimmten Regionen entnommen werden.

In einer weiteren Ausführungsform kann mit drei Bildschirmen navigiert werden, wobei drei Projektionsebenen auf einem Bildschirm vorhanden sind, auf denen die jeweilige Computertomogramm-Schicht abgebildet wird, in der sich gerade die Operationssonde befindet. Für diese Ausführungsform wird das Operationsgebiet per Computertomogramm in senkrecht aufeinander stehenden Schichten eingeteilt. Die Position der Sonde wird in drei Programme jeweils aktuell zerlegt. Dadurch ist die Position zwar auf jedem Bildschirm nur zweidimensional, für den Fachmann aber in der räumlichen Struktur definiert. Auch hier ist die Dreipunktsebene des elektronischen Kreuzes, dessen drei Fixpunkte (A,B,C) auf für mehrere aufeinanderfolgende Eingriffe in Knochen eingeschraubt ist für längere Zeit in situ verbleiben können.

Als erstes und zweites Sensorsystem und als Instrument eigen sich solche, die auf dem Markt bereits erhältlich sind, wie zum Beispiel Navigationssysteme Brain Lab (Z-Touch).

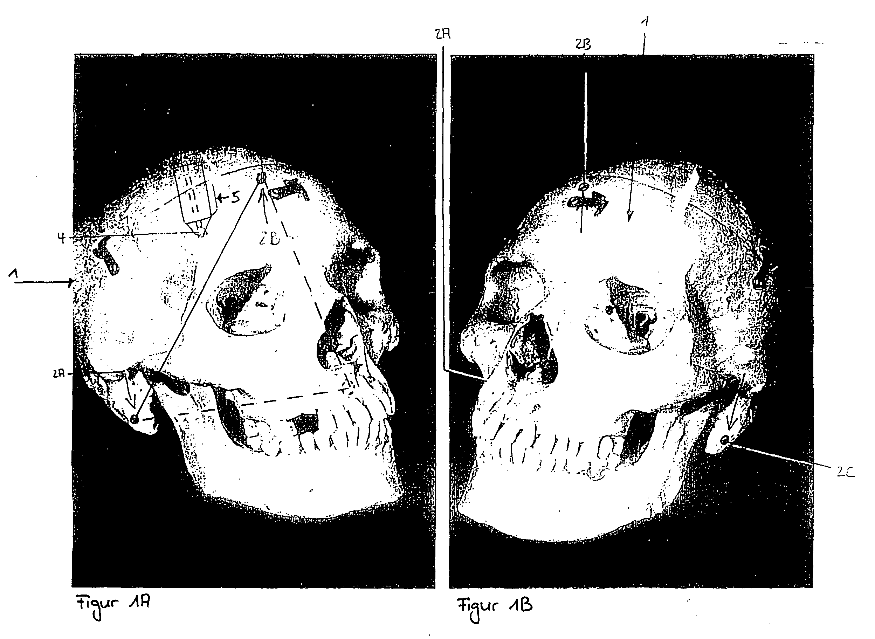

Die nachstehenden Figuren 1 a und 1b zeigen die Befestigung der Markierungen an den Fixpunkten am Gegenstand:

Figur 1a und 1b zeigen einen Schädel 1 von links respektive von rechts, bei dem an drei ersten Fixpunkten A,B und C Sender 2A, 2B und 2C durch Schrauben fixiert sind. Die Fixpunkte A und B sind die Mastoide rechts und links. Der Fixpunkt C kann fast beliebig im Bereich der Schädelkalotte gewählt werden. Während einer Operation liefern die drei Knochen verankerten Sender 2A,2B,2C die Basisdaten als fest verankertes elektronisches Referenzsystem und der Sender 4 des Instrument 5 liefert die Daten der dreidimensionalen Bewegung zum Beispiel bei einer holographischen Laseroperation. Dieses System wird mit einer holographishen Darstellung verknüpft, in die die Fixpunkte (X,Y,Z) des elektronischen Referenzsystems einprogrammiert sind und die Operationssonde fährt bei einem Eingriff im Neurokranium dreidimensional sichtbar simultan durch die Holographie. Der Operateur und seine Assistenen können nun sogar gleichzeitig aus verschiedenen Blickwinkeln die Vorgänge beobachten, sich gegenseitig vor gefählichen Strukturen warnen und Gewebsreduktionen oder die Positionierung von Medikamentendepos oder Implantaten schnell und sicher navigieren.

## Patentansprüche

1. Vorrichtung zur räumlichen Bestimmung einer Position eines Instrumentes an einem Gegenstand unter Bezugnahme auf dessen Gegenstück beziehungsweise Abbildung,
enthaltend ein erstes Sensorsystem zur Erfassung der räumlichen Lage von drei ersten Fixpunkten (A,B,C) an dem Gegenstand, ein Instrument, ein zweites Sensorsystem zur räumlichen Erfassung der Position des Instrumentes und entweder Mittel zum Abbilden des Gegenstandes oder Mittel zur Erfassung der räumlichen Lage von drei zweiten Fixpunkten (X,Y,Z) am Gegenstück und einen Zeiger,
wobei entweder das Mittel zum Abbilden des Gegenstandes den Gegenstand, drei den ersten Fixpunkten (A,B,C) entsprechende zweite Fixpunkte (X,Y,Z) und die durch das zweite Sensorsystem erfasste räumliche Position des Instrumentes abbildet,
oder das Mittel zur Erfassung der räumlichen Lage von drei zweiten Fixpunkten (X,Y,Z) am Gegenstück, wobei die zweiten Fixpunkten (X,Y,Z) den drei ersten Fixpunkten (A,B,C) des Gegenstandes entsprechen, und der Zeiger dem Anzeigen der räumlichen Position des Instrumentes am Gegenstand dient.

2. Vorrichtung gemäss Anspruch 1, enthaltend ein erstes Sensorsystem zur Erfassung der räumlichen Lage von drei ersten Fixpunkten (A,B,C) an dem Gegenstand, ein Instrument, ein zweites Sensorsystem zur räumlichen Erfassung der Position des Instrumentes und ein Mittel zum Abbilden des Gegenstandes.

3. Vorrichtung gemäss Anspruch 1, enthaltend ein erstes Sensorsystem zur Erfassung der räumlichen Lage von drei ersten Fixpunkten (A,B,C) an dem Gegenstand, ein Instrument, ein zweites Sensorsystem zur räumlichen Erfassung der Position des Instrumentes, ein Befestigungsmittel für das Gegenstück und ein Markierungsmittel.

4. Vorrichtung gemäss einem der Ansprüche 1 oder 2, in dem das Mittel zum Abbilden des Gegenstandes ein Computer ist.

5. Vorrichtung gemäss einem der Ansprüche 1 bis 4, in dem das Instrument eine Operationssonde ist.

6. Vorrichtung gemäss einem der Ansprüche 1 bis 5, in dem das erste Sensorsystem am Gegenstand fixierbare Schrauben mit Sendern enthält.

7. Vorrichtung gemäss einem der Ansprüche 1 bis 6 zur Plazierung von Gelenkersatzteilen.

8. Vorrichtung gemäss einem der Ansprüche 1 bis 6 zur Tumorentfernung.

9. Vorrichtung gemäss einem der Ansprüche 1 bis 6 zur Manipulation im Bereich der Nerven.

10. Vorrichtung gemäss einem der Ansprüche 1 bis 6 zur Manipulation im Bereich des Kopfes.

11. Verfahren zur räumlichen Bestimmung einer Position eines Instrumentes an einem Gegenstand unter Bezugnahme auf dessen Gegenstück beziehungsweise Abbildung, in dem
drei erste Fixpunkte (A,B,C) an dem Gegenstand markiert und mittels ersten Sensoren in ihrer räumlichen Lage erfasst werden,
drei zweite Fixpunkte (X,Y,Z) an dem Gegenstück beziehungsweise der Abbildung bestimmt werden, die den ersten Fixpunkten (A,B,C) entsprechen,
Orte des Gegenstandes bezüglich der ersten Fixpunkte (A,B,C) bestimmt werden, und die entsprechenden Orte des Gegenstückes beziehungsweise der Abbildung bezüglich der zweiten Fixpunkte (X,Y,Z) bestimmt werden,
die Position eines Instrumentes am Gegenstand mittels eines zweiten Sensors in seiner räumlichen Lage erfasst wird, und
die so ermittelte Position auf das Gegenstück beziehungsweise die Abbildung übertragen wird.

12. Verfahren nach Anspruch 11, in dem die Position des Instrumentes einem Ort des Gegenstandes entspricht, der bezüglich der ersten Fixpunkte (A,B,C) bestimmt wird.

13. Verfahren nach einem der Ansprüche 11 oder 12, in dem die ersten und der zweiten Sensoren die gleichen sind.

14. Verfahren nach einem der Ansprüche 11 bis 13 zur Herstellung von Prothesen.
